(19) Europäisches Patentamt
European Patent Office
Office européen des brevets

(11) **EP 1 837 347 A1**

(12) **EUROPÄISCHE PATENTANMELDUNG**

(43) Veröffentlichungstag:
**26.09.2007 Patentblatt 2007/39**

(51) Int Cl.:
**C08B 37/08** *(2006.01)*

(21) Anmeldenummer: 07005898.7

(22) Anmeldetag: **22.03.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK YU**

(30) Priorität: **22.03.2006  DE 102006013594**

(71) Anmelder: **BioPolymer GmbH & Co. KG
56410 Montabaur (DE)**

(72) Erfinder: **Laeschke, Klaus
56235 Ransbach-Baumbach (DE)**

(74) Vertreter: **Bülle, Jan et al
Kutzenberger & Wolff
Patentanwaltssozietät
Theodor-Heuss-Ring 23
50668 Köln (DE)**

(54) **Quervernetzte Gele von Hyaluronsäure und deren Verwendung**

(57)     Die vorliegenden Erfindung betrifft ein Verfahren zur Herstellung von quervernetzten Hyaluronsäure-Gelen; Hyaluronsäure-Gele, die durch dieses Verfahren erhältlich sind, und die Verwendung dieser Hyaluronsäure-Gele in kosmetischen Mitteln oder Medikamenten.

EP 1 837 347 A1

**Beschreibung**

Die vorliegende Erfindung betrifft ein Verfahren zur Herstellung von quervernetzten Hyaluronsäure-Gelen; Hyaluronsäure-Gele, die durch dieses Verfahren erhältlich sind, und die Verwendung dieser Hyaluronsäure-Gele in kosmetischen Mitteln oder Medikamenten.

Hyaluronsäure gehört zur Klasse der Gykosaminoglykane und besteht aus einem langkettigen linearen Polysaccharid. Hyaluronsäure kommt für gewöhnlich in Form des entsprechenden Natriumsalzes mit der Formel $[C_{14}H_{20}NNaO_{11}]_n$ vor, wobei n von der Herkunft der Hyaluronsäure und der Isolationsmethode abhängt.

Hyaluronsäure kann aus einer Vielzahl von Quellen isoliert werden wie beispielsweise aus der menschlichen Nabelschnur, Hahnenkämmen und dem Bindegewebe von Wirbeltieren. Die Säure ist ebenfalls ein Bestandteil von Bakterien wie Streptokokken und lässt sich daher auch durch Fermentationsprozesse erhalten.

Hyaluronsäure ist nicht immunogen und findet daher vielfältige medizinische Anwendungen, wie beispielsweise bei der Wundheilung, in der Orthopädie und in der Augenheilkunde. Ebenfalls wird Hyaluronsäure in Kosmetika verwendet.

Allerdings besitzen quervernetzte Hyaluronsäure-Gele nach der Verabreichung im menschlichen Körper bisher nur einen begrenzten Widerstand gegen den Abbau durch Enzyme wie Hyaluronidasen oder durch freie Radikale.

Eine Aufgabe der vorliegenden Erfindung war es daher, quervernetzte Hyaluronsäure-Gele zur Verfügung zu stellen, die sowohl einen hervorragenden Widerstand gegen enzymatischen Abbau als auch gegen Zersetzung durch Hydrolyse oder durch freie Radikale aufweisen, und somit insbesondere für die Verwendung *in vivo* besser geeignet sind.

Quervernetzte Hyaluronsäure-Gele besitzen die Fähigkeit zur Absorption von Wasser. Daher können diese Gele bei der Behandlung der Haut dieser Wasser entziehen. Insbesondere führt die Fähigkeit der Hyaluronsäure-Gele, Wasser zu "entziehen, bei der Behandlung von bereits geschädigter Haut zu einer weiteren Beeinträchtigung.

Eine weitere Aufgabe der vorliegenden Erfindung war es daher, quervernetzte Hyaluronsäure-Gele zur Verfügung zu stellen, die eine verringerte oder nur geringe Fähigkeit zur Absorption von Wasser besitzen, und somit für die Behandlung der Haut, insbesondere von bereits vorgeschädigter Haut, besser geeignet sind.

Ein Gegenstand der vorliegenden Erfindung ist daher ein Verfahren zur Herstellung von quervernetzten Hyaluronsäure-Gelen, wobei

1.) Hyaluronsäure in Lösung vernetzt wird,

2.) das erhaltene Hyaluronsäure-Gel filtriert und gewaschen wird,

3.) die Oberfläche des gemäß Stufe 2.) erhaltenen Hyaluronsäure-Gels vergrößert wird,

4.) und das gemäß Stufe 3.) erhaltene Hyaluronsäure-Gel in Gegenwart von Hyaluronsäure vernetzt und das so erhaltene quervernetzte Hyaluronsäure-Gel gewaschen wird.

Gemäß dem erfindungsgemäßen Verfahren können Hyaluronsäure-Gele durch einfache Verfahrensschritte und unter Verwendung von Vernetzungsmitteln, die potentiell nur wenig gesundheitsgefährdend sind, und insbesondere ohne die Verwendung von toxischen Vernetzungsmitteln, wie beispielsweise Carbodiimiden, erhalten werden. Ebenfalls ist in keinem Verfahrensschritt der Einsatz von organischen Lösungsmitteln, die potentiell umweltgefährdend sind, erforderlich.

Vorzugsweise weist die Hyaluronsäure in Stufe 1.) ein Molekulargewicht von 600.000 bis 1.800.000, besonders bevorzugt von 800.000 bis 1.600.000, ganz besonders bevorzugt von 800.000 bis 1.200.000, noch weiter bevorzugt von 900.000 bis 1.100.000 Dalton auf.

Bevorzugt liegt die Hyaluronsäure in Stufe 1.) in einer wäßrigen Lösung in einer Konzentration von 1.0 bis 3, besonders bevorzugt von 1.4 bis 3, ganz besonders bevorzugt von 1.4 bis 2.4, noch weiter bevorzugt von 1.4 bis 1.8 mg/ml vor.

Für die Zwecke der vorliegenden Erfindung ist es vorteilhaft Hyaluronsäure, ggf. in Form des entsprechenden Natriumsalzes, zu verwenden, die nicht tierischen Ursprungs ist, sonder bevorzugt durch Fermentation aus Bakterien wie beispielsweise aus Streptokokken nach den üblichen, dem Fachmann bekannten Verfahren gewonnen wird.

Vorzugsweise wird die Hyaluronsäure in Stufe 1.) durch Bildung von Ether- und/oder Ester- und/oder Sulfon- und/oder Amin- und/oder Imin- und/oder Amid-Bindungen, besonders bevorzugt durch Bildung von Ether- und/oder Ester- und/oder Sulfon-Bindungen, ganz besonders bevorzugt durch Ether- oder Sulfon-Bindungen, vernetzt.

Die Hydroxyl- und Carboxyl-Gruppen der Hyaluronsäure ermöglichen die Vernetzung, wobei Hydroxyl-Gruppen über eine Ether-Bindung und Carboxyl-Gruppen über eine Ester-Bindung vernetzt werden können. Falls notwendig, kann die Hyaluronsäure vor der Vernetzung chemisch modifiziert werden, um weitere reaktive funktionelle Gruppen einzuführen. Beispielsweise kann Hyaluronsäure durch die Behandlung mit Säuren oder Basen teilweise deacetyliert werden, so dass Amino-Gruppen zur Verfügung stehen, die wiederum über Amid-, Imin- oder Amin-Bindungen vernetzt werden können.

Die Hyaluronsäure wird in Stufe 1.) bevorzugt in Gegenwart eines Vernetzungsmittels ausgewählt aus der Gruppe bestehend aus Formaldehyd, Glutaraldehyd, Divinylsulfon, Polyanhydriden, Polyaldehyden, polyhydrischen Alkoholen, Carbodiimiden, Carbonsäurechloriden, Sulfonsäurechloriden, Epichlorhydrin, Ethylenglykol, Butandioldiglycidylether, Diglycidylether, Polyglycerolpolyglycidylether, Polyethylenglycoldiglycidylether, Polypropylengycloldiglycidylether und Bis- oder Polyepoxiden, besonders bevorzugt in Gegenwart von Butandioldiglycidylether oder Divinylsulfon, vernetzt. Butandioldiglycidylether ist dabei ein Vernetzungsmittel, das nur schwach gesundheitsgefährdend, und insbesondere viel weniger toxisch als andere Vernetzungsmittel ist.

Als weitere Bis- oder Polyepoxide seien beispielhaft Ethylenglykoldiglycidylether, (1,6)-Hexandioldiglycidylether, Polytetramethylenglykoldiglycidylether, Neopentylglykoldiglycidylether, Polyglycerolpolyglycidylether, Diglycerolpolyglycidylether, Glycerolpolyglycidylether, Trimethylolpropanpolyglycidylether, Pentaerythritolpolyglycidylether, Sorbitolpolyglycidylether, (1,2,3,4)-Diepoxybutan und (1,2,7,8)-Diepoxyoctan genannt.

Vorzugsweise wird die Vernetzung der Hyaluronsäure mittels Ether-Bindungen durch die Verwendung eines Vernetzungsmittels ausgewählt aus der Gruppe bestehend aus Formaldehyd, Glutaraldehyd und Divinylsulfon erreicht. Unter alkalischen Bedingungen, bevorzugt bei einem pH-Wert größer 10, besonders bevorzugt bei einem pH-Wert zwischen 10 und 12, können Ether-Bindungen auch durch Verwendung eines Vernetzungsmittels bevorzugt ausgewählt aus der Gruppe bestehend aus Butandioldiglycidylether, Diglycidylether, Polyglycerolpolyglycidylether, Polyethylenglycoldiglycidylether, Polypropylengycloldiglycidylether und Bis- oder Polyepoxiden, bevorzugt ausgewählt aus der Gruppe bestehend aus (1,2,3,4)-Diepoxybutan und (1,2,7,8)-Diepoxyoctan, gebildet werden.

Vorzugsweise wird die Vernetzung der Hyaluronsäure mittels Ester-Bindungen durch die Verwendung eines Vernetzungsmittels ausgewählt aus der Gruppe bestehend aus polyhydrischen Alkoholen, Carbodiimiden, Polyanhydriden und Carbonsäurechloriden erreicht. Unter sauren Bedingungen, bevorzugt bei einem pH-Wert kleiner 4, besonders bevorzugt bei einem pH-Wert zwischen 2 und 4, können Ester-Bindungen auch durch Verwendung eines Bis- oder Polyepoxids, bevorzugt ausgewählt aus der Gruppe bestehend aus (1,2,3,4)-Diepoxybutan und (1,2,7,8)-Diepoxyoctan, gebildet werden.

Die Vernetzung der Hyaluronsäure mittels Amid-Bindungen wird vorzugsweise durch die Verwendung eines Carbodiimids in Gegenwart wenigstens eines Amins, wenigstens eines Carbonsäureanhydrids oder wenigstens eines Carbonsäurechlorids im Fall von deacetylierter Hyaluronsäure und wenigstens eines Diisocyanates erreicht.

Die Vernetzung der Hyaluronsäure mittels Amin-Bindungen wird im Fall von deacetylierter Hyaluronsäure mit Amino-Gruppen vorzugsweise durch die Verwendung eines Epoxids oder die Verwendung von Glutaraldehyd in Gegenwart eines Reduktionsmittels erreicht.

Die Vernetzung der Hyaluronsäure mittels Amin-Bindungen wird im Fall von deacetylierter Hyaluronsäure mit Amino-Gruppen vorzugsweise durch die Verwendung von Glutaraldehyd erreicht.

Die Vernetzung der Hyaluronsäure mittels Sulfon-Bindungen wird vorzugsweise durch die Verwendung von Sulfonsäurechloriden erreicht.

Das Gewichtsverhältnis von Vernetzungsmittel zu Hyaluronsäure liegt vorzugsweise im Bereich von 1:10 bis 10:1.

Die Beendigung der Vernetzungsreaktion in Stufe 1.) kann durch Methoden verfolgt werden, die dem Fachmann bekannt sind.

Vorzugsweise wird das Hyaluronsäure-Gel in Stufe 2.) mit Wasser gewaschen.

Bevorzugt wird das Hyaluronsäure-Gel in Stufe 2.) über ein Siebsystem mit variabler Porengröße filtriert, so dass das Hyaluronsäure-Gel nach Filtration aus Partikeln besteht, die eine homogene Größenverteilung aufweisen. Geeignete Siebsysteme sind dem Fachmann bekannt.

Der mittlere Durchmesser der Partikel beträgt nach der Filtration 30 bis 80, bevorzugt 35 bis 70, besonders bevorzugt 40 bis 60 μm.

Für die Zwecke der vorliegenden Erfindung wird die Oberfläche des Hyaluronsäure-Gels in Stufe 3.) vorzugsweise durch Zerkleinerung, besonders bevorzugt durch Zerschlagung oder Zerteilung vergrößert. Durch die Zerkleinerung werden Partikel erhalten. Geeignete Methoden und Vorrichtungen zur Zerkleinerung sind dem Fachmann bekannt.

Die Hyaluronsäure, die in Stufe 4.) verwendet wird, kann vorzugsweise spiralförmig sein. Eine entsprechende Hyaluronsäure kann beispielsweise von der Firma HTL SAS (Fougeres, Frankreich) bezogen werden.

Bevorzugt weist die Hyaluronsäure in Stufe 4.) ein Molekulargewicht von 600.000 bis 1.800.000, besonders bevorzugt von 800.000 bis 1.600.000, ganz besonders bevorzugt von 800.000 bis 1.200.000, noch weiter bevorzugt von 900.000 bis 1.100.000 Dalton auf.

Bevorzugt wird die Hyaluronsäure in Stufe 4.) in einer wäßrigen Lösung in einer Konzentration von 1.0 bis 3, besonders bevorzugt von 1.4 bis 3, ganz besonders bevorzugt von 1.4 bis 2.4, noch weiter bevorzugt von 1.4 bis 1.8 mg/ml zugegeben.

Die entsprechende Lösung der Hyaluronsäure in Stufe 4.) weist vorzugsweise einen pH-Wert von 6.9 bis 7.8 auf.

Vorzugsweise wird das Hyaluronsäure-Gel in Stufe 4.) durch Bildung von Ether- und/oder Ester- und/oder Sulfon- und/oder Amin- und/oder Imin- und/oder Amid-Bindungen, besonders bevorzugt durch Bildung von Ether- und/oder Ester- und/oder Sulfon-Bindungen, ganz besonders bevorzugt durch Ether- oder Sulfon-Bindungen, in Gegenwart von Hyaluronsäure vernetzt.

Das Hyaluronsäure-Gel wird in Stufe 4.) in Gegenwart eines Vernetzungsmittels bevorzugt ausgewählt aus der Gruppe bestehend aus Formaldehyd, Glutaraldehyd, Divinylsulfon, Polyanhydriden, Polyaldehyden, polyhydrischen Alkoholen, Carbodiimiden, Carbonsäurechloriden, Sulfonsäurechloriden, Epichlorhydrin, Ethylenglykol, Butandioldiglycidylether, Diglycidylether, Polyglycerolpolyglycidylether, Polyethylenglycoldiglycidylether, Polypropylengcloldiglycidylether und Bis- oder Polyepoxiden, besonders bevorzugt in Gegenwart von Butandioldiglycidylether oder Divinylsulfon, in Gegenwart Hyaluronsäure vernetzt.

Bevorzugt wird das quervernetzte Hyaluronsäure-Gel in Stufe 4.) mit Wasser gewaschen.

Ein weiterer Gegenstand der vorliegenden Erfindung ist ein Hyaluronsäure-Gel wie es nach dem vorstehend beschriebenem Verfahren erhältlich ist.

Bevorzugt beträgt der Vernetzungsgrad des erfindungsgemäßen Hyaluronsäure-Gels > 95 %, besonders bevorzugt > 99 %, ganz besonders bevorzugt > 99.5 %, noch weiter bevorzugt nahezu 100 %.
Vorzugsweise beträgt die Viskosität des erfindungsgemäßen Hyaluronsäure-Gels 4000 bis 25000, besonders bevorzugt 4500 bis 22000, ganz besonders bevorzugt 5000 bis 20000, noch weiter bevorzugt 5500 bis 18000, am weitesten bevorzugt 6000 bis 16000 mPa•s.

Die Viskosität kann mit einem Rotationsviskosimeter vom Typ Rheometer RS1 bei einer Temperatur von 20 °C bei 10 Umdrehungen pro Sekunde bestimmt werden.

Die erfindungsgemäßen Hyaluronsäure-Gele finden eine Vielzahl pharmazeutischer, medizinischer, insbesondere chirurgischer, und kosmetischer Anwendungen.

Daher ist ein weiterer Gegenstand der vorliegenden Anmeldung ein kosmetisches Mittel umfassend wenigstens ein quervernetztes Hyaluronsäure-Gel wie es nach dem vorstehend beschriebenem Verfahren erhältlich ist und einen oder mehrere kosmetische Hilfs- und Trägerstoffe.

Vorzugsweise werden kosmetischen Hilfs- und Trägerstoffe ausgewählt aus der Gruppe bestehend aus Wasser, Konservierungsmitteln, Farbstoffen, Pigmenten, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren, Radikalfänger, Vitamine, Lichtschutzfilter, Enzyme, Melanin und Antioxidationsmittel.

Besonders bevorzugt ist ein kosmetisches Mittel umfassend wenigstens ein Hyaluronsäure-Gel wie es nach dem vorstehend beschriebenem Verfahren erhältlich ist, wenigstens ein Konservierungsmittel und Wasser.

Bevorzugt wird das Konservierungsmittel ausgewählt aus der Gruppe bestehend aus Natriumbenzoat, Poly-3-hydroxybutyrat-methylester und Poly-3-hydroxybutyratpropylester.

Für den Fall des Einsatzes von Lichtschutzfiltern seien beispielhaft wasserlösliche UVB-Filter, beispielsweise Sulfonsäurederivate von Benzophenon oder von 3-Benzylidencampher oder Salze wie das Na- oder K-Salz der 2-Phenylbenzimidazol-5-sulfonsäure genannt.

Beispielhaft als Antioxidationsmitteln seien Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z. B alpha-Carotin, beta-Carotin; Harnsäure und Derivate davon; alpha-Hydroxysäuren wie Citronensäure, Milchsäure, Apfelsäure; Stilbene und deren Derivate; sowie Granatapfelextrakte genannt.

Das erfindungsgemäße kosmetische Mittel kann als Emulsion oder Gel vorliegen.

Die für eine Emulsion eingesetzten Öle können konventionelle kosmetische Öle sein, beispielsweise Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Mineralöle, Polyisopren, Squalane, Trimethylpropantriisostearat, Isodecylcitrat, Neopentylglycol-diheptanoat, PPG-15-stearylether sowie pflanzliche Öle, wie Calendulaöl, Jojobaöl, Avocadoöl, Macadamianussöl, Rizinusöl, Weizenkeimöl, Traubenkernöl, Kukuinussöl, Distelöl, Nachtkerzenöl, Safloröl oder ein Gemisch mehrerer davon.

Beispielhaft als Gelbildner für ein kosmetisches Gel seien Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon und Montmorillonit genannt.

Als Hilfsstoffe verwendete Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile und verkapselte und unverkapselte Getreidestärken.

Eine besondere Wirkung der erfindungsgemäßen kosmetischen Mittels besteht in seiner langanhaltenden feuchtigkeitsspendenden Wirkung.

Die Verwendung des erfindungsgemäßen kosmetischen Mittel kann beispielsweise in Form von Sonnencremes, Sonnengelen, After-sun-Produkten, Tagescremes, Nachtcremes, Masken, Körperlotionen, Reinigungsmilch, Körperpuder, Augenkosmetik, Haarmasken, Haarspülungen, Haarshampoos, Badeölen und in Produkten der dekorativen Kosmetik wie Deo-Stiften, Parfüm-Stiften, Lippenstiften, Gelen, Lidschatten, Kompaktprodukten wie Kompaktpuder oder Kompaktwachs, Rouge, Grundierung und Make-up erfolgen. Die Herstellung derartiger Produkte erfolgt auf eine Weise, wie sie dem Fachmann auf diesem Gebiet bekannt ist.

Ein weiterer Gegenstand der vorliegenden Erfindung ist die Verwendung wenigstens eines quervernetzten Hyaluronsäure-Gels wie es nach dem vorstehend beschriebenem Verfahren erhältlich ist in kosmetischen Mitteln.

Ebenfalls ein Gegenstand der vorliegenden Erfindung ist ein Medikament enthaltend wenigstens ein quervernetztes Hyaluronsäure-Gel wie es nach dem vorstehend ,beschriebenem Verfahren erhältlich ist und ggf. einen oder mehrere pharmazeutische Hilfsmittel.

Als pharmazeutischen Hilfsstoffe seien beispielsweise Lösungsmittel, beispielsweise wässrige Pufferlösung, Lösungsvermittler, Lösungsbeschleuniger, Salze, Salzbildner, Puffersubstanzen, Viskositätsregler, Konsistenzbeeinflusser wie Verdickungsmittel, Sedimentationsverzögerer, Antioxidantien, Konservierungsmittel, Mittel zur Isotonisierung, Tenside (Emulgatoren, Solubilisatoren, Benetzer, Entschäumer, Spreitmittel, Geruchs- und Geschmackskorrigentien und Resorptionsbeschleuniger genannt.

Ein Viskositätsregler zur Einstellung einer gewünschten Viskosität kann vorzugsweise aus der Gruppe bestehend aus Chondroitinsulfat, Polyvinylpyrrolidon, Polyvinylalkohol, Polyacrylsäure, Polyacrylamid, Polyacrylharze, Polyethylenglykol, Cellulosederivate sowie Mischungen davon ausgewählt werden. Als geeignete Cellulosederivate können beispielsweise Hydroxyethylcellulose, Hydroxypropylmethylcellulose und Carboxymethylcellulose verwendet werden.

Bevorzugt eignet sich das erfindungsgemäße Medikament zur Behandlung und/oder Prophylaxe ophthalmologischer und/oder rhinologischer Fehlfunktionen, zur Hautbehandlung, bevorzugt zur Wundheilung oder Behandlung von Falten, insbesondere Faltenunterspritzung, zur Behandlung von Gewebedefekten, zur Behandlung und/oder Prophylaxe von Stressinkontinenz und/oder Stressharninkontinenz, zur Behandlung und/oder Prophylaxe von interstitieller Cystitis oder zur Behandlung und/oder Prophylaxe von Gonarthrose und/oder Gonarthritis.

Die ophthalmologische Fehlfunktion kann bevorzugt aus der Gruppe bestehend aus Rhinitis allergica, allergischer Rhinokonjunktivitis, allergischer Keratokonjunktivitis, atopischer Keratokonjunktivitis, gigantopapilläre Konjunktivitis, Episkleritis, Skleritis, Tenonitis, Sjögren-Syndrom, Conjunctivitis vernalis und Mischformen davon ausgewählt werden.

Die rhinologische Fehlfunktion kann vorzugsweise aus der Gruppe bestehend aus chronischer Rhinitis, Rhinitis sicca und Mischformen davon ausgewählt werden.

Besonders bevorzugt ist die Verwendung des erfindungsgemäßen Gels zur Behandlung von Falten, insbesondere zur Faltenunterspritzung.

Die erfindungsgemäßen Hyaluronsäure-Gele können ebenfalls dazu genutzt werden, das Zusammenwachsen von Organen nach operativen Eingriffen zu vermeiden oder zumindest zu reduzieren.

Ferner können die erfindungsgemäßen Hyaluronsäure-Gele in der Chirurgie als Implantate verwendet werden, beispielsweise als Ersatz von Knorpel und/oder Knochen oder als Brustimplantate. Die Hyaluronsäure-Gele lassen sich ebenfalls als Beschichtung für Implantate verwenden, die dauerhaft im Körper verwendet werden, wie beispielsweise Brustimplantate, Katheter, Kanülen, Knochenprothesen, Knorpelersatz, Minipumpen und andere Wirkstoffabgabesysteme und künstliche Organe und Blutgefäße. Sie können ebenfalls als Gleitmittel bei der Behandlung von Arthritis verwendet werden.

Eine weitere Verwendung der erfindungsgemäßen Hyaluronsäure-Gele besteht in der Abgabe von Wirkstoffen. Geeignete Wirkstoffe in diesem Zusammenhang umfassen Chemotherapeutika, entzündungshemmende Wirkstoffe, Antibiotika, Analgetika, Anaesthetika, zytostatische Wirkstoffe, Immunostimulantien und Wirkstoffe, die die Wundheilung unterstützen. Die Wirkstoffe können dabei durch Methoden, die dem Fachmann bekannt sind, an das Hyaluronsäure-Gel gebunden werden.

Je nach Art der gewünschten Anwendung kann das erfindungsgemäße Medikament als Injektionspräparat, Lösung, insbesondere Spüllösung und Infusionslösung, Suspension, Emulsion, Gel, Salbe, Paste oder in Form von Pulvern, Pellets, Granulaten, Kapseln und Tabletten formuliert werden.

Bevorzugt kann ein erfindungsgemäßes Medikament zur Hautbehandlung oder zur Behandlung von Gewebedefekten als Injektionspräparat, Gel, Salbe oder Paste formuliert werden, die jeweils übliche, dem Fachmann bekannte Hilfsstoffe enthalten können.

Vorzugweise kann ein erfindungsgemäßes Medikament zur Behandlung und/oder Prophylaxe ophthalmologischer Fehlfunktionen in Form einer Lösung vorliegen, so dass dieses beispielsweise in Form von Augentropfen oder in Form eines Augensprays auf die Hornhautoberfläche des Auges aufgebracht werden kann.

Selbstverständlich ist es möglich, dass das erfindungsgemäße Medikament zur Behandlung und/oder Prophylaxe ophthalmologischer Fehlfunktionen in der Form eines Feststoffes vorliegt, der vor einer Applikation zunächst in einer wässrigen Lösung wie beispielsweise einer Pufferlösung aufgelöst wird. Nach Auflösung eines Feststoffes, beispielsweise in einer wässrigen Pufferlösung, wird diese Lösung sterilfiltriert und kann dann als Augenspray bzw. Augentropfen auf die Cornea aufgebracht werden. Bevorzugt liegen Feststoff und Lösungsmittel bei getrennter Aufbewahrung bereits in steriler Form vor, so dass ein Sterilfiltrieren nach Herstellung der Lösung nicht erforderlich ist. Der Anwender kann somit nach Herstellung der Mischung bzw. Lösung die pharmazeutische Zusammensetzung direkt applizieren. Ebenfalls kann das erfindungsgemäße Medikament auch in Form von Augentabletten in den Bindehautsack eingebracht werden oder in Form von Augensalben bzw. Augengelen verwendet werden.

Ein weiterer Gegenstand der vorliegenden Erfindung ist die Verwendung wenigstens eines quervernetzten Hyaluronsäure-Gels wie es nach dem vorstehend beschriebenem Verfahren erhältlich ist zur Herstellung eines Medikaments zur Behandlung und/oder Prophylaxe ophthalmologischer und/oder rhinologischer allergischer Komplikationen oder zur Hautbehandlung, bevorzugt zur Wundheilung oder Behandlung von Falten, zur Behandlung von Gewebedefekten, zur Behandlung und/oder Prophylaxe von Stressinkontinenz und/oder Stressharninkontinenz, zur Behandlung und/oder Prophylaxe von interstitieller Cystitis oder zur Behandlung und/oder Prophylaxe von Gonarthrose und/oder Gonarthritis.

Die ophthalmologische Fehlfunktion wird bevorzugt aus der Gruppe bestehend aus Rhinitis allergica, allergischer Rhinokonjunktivitis, allergischer Keratokonjunktivitis, atopischer Keratokonjunktivitis, gigantopapilläre Konjunktivitis, Episkleritis, Skleritis, Tenonitis, Sjögren-Syndrom, Conjunctivitis vernalis und Mischformen davon ausgewählt. Die rhinologische Fehlfunktion wird vorzugsweise aus der Gruppe bestehend aus chronischer Rhinitis, Rhinitis sicca und Mischformen davon ausgewählt.

**Methoden:**

**1. Bestimmung der Wasserabsorptionsfähigkeit**

20 mg (Wd) getrocknetes quervernetztes Hyaluronsäure-Gel werden für 24 Stunden in PBS Pufferlösung eingetaucht, um ein geschwollenes Gel zu erhalten. Das feuchte Gel wird abfiltriert und Rückstände von Wasser an dessen Oberfläche werden mit Tissuepapier entfernt. Das Gewicht des feuchten Gels wird bestimmt (Ws). Die Wasserabsorptionsfähigkeit (WAC in %) ergibt sich damit aus der Gleichung:

$$WAC\ (\%) = (Ws\text{-}Wd)/Wd \times 100$$

**2. Widerstand gegen Verdauung durch Hyaluronidase**

20 mg quervernetztes Hyaluronsäure-Gel werden in PBS Pufferlösung (6 ml, pH-Wert 7.2) suspendiert, mit Hyaluronidase (1000 U) versetzt und für 24 Stunden bei 37 °C inkubiert. Das Gel wird entfernt und mit PBS Pufferlösung gewaschen, so dass 10 mL einer Lösung erhalten werden. Die Lösung wird 30 Minuten gekocht und für 10 Minuten bei 4000 Umdrehungen zentrifugiert. Die überstehende Flüssigkeit wird mit PBS Pufferlösung auf 25 mL erhöht und die Konzentration der Hyaluronsäure wird mit Hilfe des Carbazol-Assays bestimmt.

Der Gewichtsverlust des Hyaluronsäure-Gels, der aufgrund der Verdauung mit Hyaluronidase auftritt, kann mit Hilfe der folgenden Gleichung bestimmt werden:

$$\text{Gewichtsverlust Hyaluronsäure-Gel (\%)} = ([HA] \times 25/\ HA_0) \times 100$$

wobei [HA] die Konzentration der Hyaluronsäure in Lösung und $HA_0$ die Menge an Hyaluronsäure im quervernetzten Hyaluronsäure-Gel angibt.

**3. Widerstand gegen den Abbau durch freie Radikale**

Fenton-Reagenz, das aus 25 $\mu$L 0.1 M Ascorbinsäure und 0.25 $\mu$L 0.1 M Wasserstoffperoxid in 5 mL PBS Pufferlösung hergestellt wird, wird zur Darstellung freier Radikale verwendet. 20 mg quervernetztes Hyaluronsäure-Gel werden zu dieser Lösung gegeben und die Lösung wird für 24 Stunden bei 37 °C inkubiert.

Das Gel wird entfernt und mit PBS Pufferlösung gewaschen, so dass 25 mL einer Lösung erhalten werden. Die Konzentration der Hyaluronsäure wird mit Hilfe des Carbazol-Assays bestimmt.

Der Gewichtsverlust des Hyaluronsäure-Gels wird mit Hilfe der unter 2. angegebenen Formel bestimmt.

**Patentansprüche**

1. Verfahren zur Herstellung von quervernetztem Hyaluronsäure-Gel, wobei

    1.) Hyaluronsäure in Lösung vernetzt wird,
    2.) das erhaltene Hyaluronsäure-Gel filtriert und gewaschen wird,
    3.) die Oberfläche des gemäß Stufe 2.) erhaltenen Hyaluronsäure-Gels vergrößert wird,
    4.) das gemäß Stufe 3.) erhaltene Hyaluronsäure-Gel in Gegenwart von Hyaluronsäure vernetzt wird und das so erhaltene quervernetzte Hyaluronsäure-Gel gewaschen wird.

2. Verfahren gemäß Anspruch 1, **dadurch gekennzeichnet, dass** die Hyaluronsäure in Stufe 1.) ein Molekulargewicht von 600.000 bis 1.800.000, bevorzugt von 800.000 bis 1.600.000, besonders bevorzugt von 800.000 bis 1.200.000 Dalton, ganz besonders bevorzugt von 900.000 bis 1.100.000 Dalton aufweist.

3. Verfahren gemäß Anspruch 1 oder 2, **dadurch gekennzeichnet, dass** die Konzentration an Hyaluronsäure in Lösung in Stufe 1.) 1.0 bis 3, bevorzugt 1.4 bis 3, besonders bevorzugt 1.4 bis 2.4, ganz besonders bevorzugt 1.4 bis 1.8 mg/ml beträgt.

4. Verfahren gemäß einem oder mehreren der Ansprüche 1 bis 4, **dadurch gekennzeichnet, dass** die Hyaluronsäure in Stufe 1.) in einer wässrigen Lösung vorliegt.

5. Verfahren gemäß einem oder mehreren der Ansprüche 1 bis 3, **dadurch gekennzeichnet, dass** die Hyaluronsäure in Stufe 1.) durch Bildung von Ether-und/oder Ester- und/oder Sulfon- und/oder Amin- und/oder Imin- und/oder Amid-Bindungen, bevorzugt durch Bildung von Ether- und/oder Ester- und/oder Sulfon-Bindungen, besonders bevorzugt durch Ether- oder Sulfon-Bindungen, vernetzt wird.

6. Verfahren gemäß einem oder mehreren der Ansprüche 1 bis 5, **dadurch gekennzeichnet, dass** die Hyaluronsäure in Stufe 1.) in Gegenwart eines Vernetzungsmittels ausgewählt aus der Gruppe bestehend aus Formaldehyd, Glutaraldehyd, Divinylsulfon, Polyanhydriden, Polyaldehyden, polyhydrischen Alkoholen, Carbodiimiden, Carbonsäurechloriden, Sulfonsäurechloriden, Epichlorhydrin, Ethylenglykol, Butandioldiglycidylether, Diglycidylether, Polyglycerolpolyglycidylether, Polyethylenglycoldiglycidylether, Polypropylengycloldiglycidylether und Bis- oder Polyepoxiden, bevorzugt in Gegenwart von Butandioldiglycidylether oder Divinylsulfon, vernetzt wird.

7. Verfahren gemäß einem oder mehreren der Ansprüche 1 bis 6, **dadurch gekennzeichnet, dass** das Hyaluronsäure-Gel in Stufe 2.) mit Wasser gewaschen wird.

8. Verfahren gemäß einem oder mehreren der Ansprüche 1 bis 7, **dadurch gekennzeichnet, dass** das Hyaluronsäure-Gel in Stufe 2.) über ein Siebsystem mit variabler Porengröße filtriert wird.

9. Verfahren gemäß einem oder mehreren der Ansprüche 1 bis 8, **dadurch gekennzeichnet, dass** das Hyaluronsäure-Gel in Stufe 2.) nach Filtration aus Partikeln von homogener Größenverteilung besteht.

10. Verfahren gemäß Anspruch 9, **dadurch gekennzeichnet, dass** der mittlere Durchmesser der Partikel 30 bis 80, bevorzugt 35 bis 70, besonders bevorzugt 40 bis 60 $\mu$m beträgt.

11. Verfahren gemäß einem oder mehreren der Ansprüche 1 bis 10, **dadurch gekennzeichnet, dass** die Oberfläche des Hyaluronsäure-Gels in Stufe 3.) durch Zerkleinerung vergrößert wird.

12. Verfahren gemäß einem oder mehreren der Ansprüche 1 bis 11, **dadurch gekennzeichnet, dass** die Hyaluronsäure in Stufe 4.) ein Molekulargewicht von 600.000 bis 1.800.000, bevorzugt von 800.000 bis 1.600.000, besonders bevorzugt von 800.000 bis 1.200.000, ganz besonders bevorzugt von 900.000 bis 1.100.000 Dalton aufweist.

**13.** Verfahren gemäß einem oder mehreren der Ansprüche 1 bis 12 , **dadurch gekennzeichnet, dass** die Hyaluronsäure in Stufe 4.) in einer Lösung mit einer Konzentration von 1.0 bis 3, bevorzugt 1.4 bis 3, besonders bevorzugt von 1.4 bis 2.4, ganz besonders bevorzugt von 1.4 bis 1.8 mg/ml zugegeben wird.

**14.** Verfahren gemäß Anspruch 13, **dadurch gekennzeichnet, dass** die Hyaluronsäure in Stufe 4.) in einer wässrigen Lösung vorliegt.

**15.** Verfahren gemäß Anspruch 13 oder 14, **dadurch gekennzeichnet, dass** die Lösung der Hyaluronsäure in Stufe 4.) einen pH-Wert von 6.9 bis 7.8 aufweist.

**16.** Verfahren gemäß einem oder mehreren der Ansprüche 1 bis 15, **dadurch gekennzeichnet, dass** das Hyaluronsäure-Gel in Stufe 4.) durch Bildung von Ether- und/oder Ester- und/oder Sulfon- und/oder Amin- und/oder Imin- und/oder Amid-Bindungen, bevorzugt durch Bildung von Ester- und/oder Ether- und/oder Sulfon-Bindungen, besonders bevorzugt Ether- oder Sulfon-Bindungen, in Gegenwart von Hyaluronsäure vernetzt wird.

**17.** Verfahren gemäß einem oder mehreren der Ansprüche 1 bis 16, **dadurch gekennzeichnet, dass** das Hyaluronsäure-Gel in Stufe 4.) in Gegenwart eines Vernetzungsmittels ausgewählt aus der Gruppe bestehend aus Formaldehyd, Glutaraldehyd, Divinylsulfon, Polyanhydriden, Polyaldehyden, polyhydrischen Alkoholen, Carbodiimiden, Carbonsäurechloriden, Sulfonsäurechloriden, Epichlorhydrin, Ethylenglykol, Butandioldiglycidylether, Diglycidylether, Polyglycerolpolyglycidylether, Polyethylenglycoldiglycidylether, Polypropylengycloldiglycidylether und Bis- oder Polyepoxiden, bevorzugt in Gegenwart von Butandioldiglycidylether oder Divinylsulfon, in Gegenwart von Hyaluronsäure vernetzt wird.

**18.** Verfahren gemäß Anspruch einem oder mehreren der Ansprüche 1 bis 17, **dadurch gekennzeichnet, dass** das quervernetzte Hyaluronsäure-Gel in Stufe 4.) mit Wasser gewaschen wird.

**19.** Verfahren nach einem oder mehreren der Ansprüche 1 bis 18, **dadurch gekennzeichnet, dass** der Vernetzungsgrad des gemäß Stufe 4.) erhaltenen quervernetzten Hyaluronsäure-Gels > 95 %, bevorzugt > 99 %, besonders bevorzugt > 99.5 %, ganz besonders bevorzugt nahezu 100 % beträgt.

**20.** Verfahren nach einem oder mehreren der Ansprüche 1 bis 19, **dadurch gekennzeichnet, dass** die Viskosität des gemäß Stufe 4.) erhaltenen quervernetzten Hyaluronsäure-Gels 4000 bis 25000, bevorzugt 4500 bis 22000, besonders bevorzugt 5000 bis 20000, ganz besonders bevorzugt 5500 bis 18000, noch weiter bevorzugt 6000 bis 16000 mPa•s beträgt.

**21.** Quervernetztes Hyaluronsäure-Gel erhältlich gemäß dem Verfahren nach einem oder mehreren der Ansprüche 1 bis 20.

**22.** Kosmetisches Mittel umfassend wenigstens ein quervernetztes Hyaluronsäure-Gel gemäß Anspruch 21 und einen oder mehrere kosmetische Hilfs- und Trägerstoffe.

**23.** Kosmetisches Mittel gemäß Anspruch 22, **dadurch gekennzeichnet, dass** die kosmetischen Hilfs- und Trägerstoffe ausgewählt sind aus der Gruppe bestehend aus Wasser, Konservierungsmitteln, Farbstoffen, Pigmenten, Verdikkungsmittel, Duftstoffe, Alkohole, Polyole, Ester, Elektrolyte, Gelbildner, polare und unpolare Öle, Polymere, Copolymere, Emulgatoren, Wachse, Stabilisatoren, Radikalfänger, Vitamine, Lichtschutzfilter, Enzyme, Melanin und Antioxidationsmittel.

**24.** Kosmetisches Mittel gemäß Anspruch 22 oder 23 umfassend wenigstens ein Hyaluronsäure-Gel gemäß Anspruch 21, wenigstens ein Konservierungsmittel und Wasser.

**25.** Kosmetisches Mittel gemäß Anspruch 23 oder 24, **dadurch gekennzeichnet, dass** das Konservierungsmittel ausgewählt wird aus der Gruppe bestehend aus Natriumbenzoat, Poly-3-hydroxybutyrat-methylester und Poly-3-hydroxybutyrat-propylester.

**26.** Verwendung wenigstens eines quervernetzten Hyaluronsäure-Gels gemäß Anspruch 21 in kosmetischen Mitteln.

**27.** Medikament enthaltend wenigstens ein quervernetztes Hyaluronsäure-Gel gemäß Anspruch 21 und ggf. einen oder mehrere pharmazeutische Hilfsmittel.

**28.** Medikament gemäß Anspruch 27 zur Behandlung und/oder Prophylaxe ophthalmologischer und/oder rhinologischer Fehlfunktionen, zur Hautbehandlung, bevorzugt zur Wundheilung oder Behandlung von Falten, zur Behandlung von Gewebedefekten, zur Behandlung und/oder Prophylaxe von Stressinkontinenz und/oder Stressharninkontinenz, zur Behandlung und/oder Prophylaxe von interstitieller Cystitis oder zur Behandlung und/oder Prophylaxe von Gonarthrose und/oder Gonarthritis.

**29.** Medikament gemäß Anspruch 28, **dadurch gekennzeichnet, dass** die ophthalmologische Fehlfunktion aus der Gruppe bestehend aus Rhinitis allergica, allergischer Rhinokonjunktivitis, allergischer Keratokonjunktivitis, atopischer Keratokonjunktivitis, gigantopapilläre Konjunktivitis, Episkleritis, Skleritis, Tenonitis, Sjögren-Syndrom, Conjunctivitis vernalis und Mischformen davon ausgewählt ist.

**30.** Medikament gemäß Anspruch 28, **dadurch gekennzeichnet, dass** die rhinologische Fehlfunktion aus der Gruppe bestehend aus chronischer Rhinitis, Rhinitis sicca und Mischformen davon ausgewählt wird.

**31.** Verwendung wenigstens eines Hyaluronsäure-Gels gemäß Anspruch 21 zur Herstellung eines Medikaments zur Behandlung und/oder Prophylaxe ophthalmologischer und/oder rhinologischer allergischer Komplikationen oder zur Hautbehandlung, bevorzugt zur Wundheilung oder Behandlung von Falten, zur Behandlung von Gewebedefekten, zur Behandlung und/oder Prophylaxe von Stressinkontinenz und/oder Stressharninkontinenz, zur Behandlung und/oder Prophylaxe von interstitieller Cystitis oder zur Behandlung und/oder Prophylaxe von Gonarthrose und/oder Gonarthritis.

**32.** Verwendung gemäß Anspruch 31, **dadurch gekennzeichnet, dass** die allergischen Komplikation aus der Gruppe bestehend aus Rhinitis allergica, allergischer Rhinokonjunktivitis, allergischer Keratokonjunktivitis, atopischer Keratokonjunktivitis, gigantopapilläre Konjunktivitis, Episkleritis, Skleritis, Tenonitis, Sjögren-Syndrom, Conjunctivitis vernalis und Mischformen davon ausgewählt wird.

**33.** Verwendung gemäß Anspruch 31, **dadurch gekennzeichnet, dass** die rhinologische Fehlfunktion aus der Gruppe bestehend aus chronischer Rhinitis, Rhinitis sicca und Mischformen davon ausgewählt wird.

Europäisches
Patentamt

**EUROPÄISCHER RECHERCHENBERICHT**

Nummer der Anmeldung

EP 07 00 5898

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/127698 A1 (TSAI SHIAO-WEN [TW] ET AL) 1. Juli 2004 (2004-07-01) | 1-22,26, 27 | INV. C08B37/08 |
| Y | * Absätze [0025], [0026] * | 23-25, 28-33 | |
| | * Absätze [0033], [0036], [0037], [0040], [0043], [0045], [0046] * * Beispiele 5-7 * ----- | | |
| Y | WO 00/46252 A (FERMENTECH MED LTD [GB]; ZHAO XIAOBIN [GB]; ALEXANDER CATHERINE [GB];) 10. August 2000 (2000-08-10) * Seite 4, Zeile 19 - Seite 9, Zeile 28 * ----- | 23-25, 28-33 | |
| A | WO 97/04012 A (AAGERUP BENGT [SE]) 6. Februar 1997 (1997-02-06) * Seite 4, Zeile 21 - Seite 5, Zeile 17 * * Seite 6, Zeilen 5-13 * * Seite 7, Zeilen 1-12,23-36 * * Seite 8, Zeilen 18-22 * ----- | 1-33 | |

RECHERCHIERTE SACHGEBIETE (IPC)

C08B

Der vorliegende Recherchenbericht wurde für alle Patentansprüche erstellt

| Recherchenort | Abschlußdatum der Recherche | Prüfer |
|---|---|---|
| München | 26. April 2007 | Gerber, Myriam |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
........................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 07 00 5898

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-04-2007

| Im Recherchenbericht angeführtes Patentdokument | Datum der Veröffentlichung | Mitglied(er) der Patentfamilie | | Datum der Veröffentlichung |
|---|---|---|---|---|
| US 2004127698 A1 | 01-07-2004 | TW | 251596 B | 21-03-2006 |
| | | US | 2007066816 A1 | 22-03-2007 |
| WO 0046252 A | 10-08-2000 | AT | 335011 T | 15-08-2006 |
| | | AU | 773628 B2 | 27-05-2004 |
| | | AU | 2307200 A | 25-08-2000 |
| | | BR | 0007985 A | 06-11-2001 |
| | | CA | 2362233 A1 | 10-08-2000 |
| | | CN | 1342170 A | 27-03-2002 |
| | | DE | 1163274 T1 | 14-11-2002 |
| | | DK | 1163274 T3 | 04-12-2006 |
| | | EP | 1163274 A1 | 19-12-2001 |
| | | ES | 2181607 T1 | 01-03-2003 |
| | | JP | 2002536465 T | 29-10-2002 |
| | | MX | PA01007877 A | 04-06-2003 |
| | | NZ | 512994 A | 28-11-2003 |
| | | US | 2002049281 A1 | 25-04-2002 |
| WO 9704012 A | 06-02-1997 | AT | 204000 T | 15-08-2001 |
| | | AU | 700215 B2 | 24-12-1998 |
| | | AU | 6371896 A | 18-02-1997 |
| | | BR | 9609534 A | 23-02-1999 |
| | | CN | 1190974 A | 19-08-1998 |
| | | CZ | 9800129 A3 | 17-06-1998 |
| | | DE | 69614391 D1 | 13-09-2001 |
| | | DE | 69614391 T2 | 14-02-2002 |
| | | DE | 839159 T1 | 07-10-1999 |
| | | DK | 839159 T3 | 08-10-2001 |
| | | EA | 980133 A1 | 29-10-1998 |
| | | EP | 0839159 A1 | 06-05-1998 |
| | | ES | 2161368 T3 | 01-12-2001 |
| | | GR | 3037065 T3 | 31-01-2002 |
| | | HU | 9901714 A2 | 28-09-1999 |
| | | JP | 11509256 T | 17-08-1999 |
| | | JP | 3094074 B2 | 03-10-2000 |
| | | NO | 980213 A | 16-03-1998 |
| | | NZ | 312229 A | 25-11-1998 |
| | | PL | 324608 A1 | 08-06-1998 |
| | | PT | 839159 T | 30-11-2001 |
| | | SK | 6198 A3 | 11-01-1999 |
| | | TR | 9800072 T1 | 21-05-1998 |
| | | US | 5827937 A | 27-10-1998 |

EPO FORM P0461

Für nähere Einzelheiten zu diesem Anhang : siehe Amtsblatt des Europäischen Patentamts, Nr.12/82